# EUROPÄISCHE PATENTANMELDUNG

(11) **EP 1 908 438 A1**
(43) Veröffentlichungstag der Anmeldung: **09.04.2008**
(21) Anmeldenummer: 06121632.1
(22) Anmeldetag: 02.10.2006
(51) Int. Cl.: A61F 5/08

(54) **Vorrichtung zum Umformen von Knochen**

(71) Anmelder: Honegger, Norina, 8704 Herrliberg (CH); Bauer, Erwin, 8008 Zürich (CH)
(72) Erfinder: Honegger, Norina, 8704 Herrliberg (CH); Bauer, Erwin, 8008 Zürich (CH)
(74) Vertreter: Dr. Graf & Partner

(57) **Zusammenfassung**

Die Vorrichtung (1) zum Umformen von Knochen, insbesondere Nasenknochen, umfasst:
eine Formvorrichtung (2) mit einer zur Auflage auf die Haut bestimmten Anpressfläche (2d),
und eine Haltevorrichtung (3), welche mit der Formvorrichtung (2) verbindbar ist,

wobei die Haltevorrichtung (3) sowie die Formvorrichtung (2) derart zusammenwirkend ausgestaltet ist, dass die Formvorrichtung (2) zumindest an der Anpressfläche (2d) unter Ausübung einer Anpresskraft an der Haut anliegen kann.

## Beschreibung

Die Erfindung betrifft eine Vorrichtung zum Umformen von Knochen beziehungsweise zur Beeinflussung des Knochenwachstums, insbesondere des Nasenbeins. Die Erfindung betrifft weiter ein Verfahren zum Herstellen einer Vorrichtung zum Umformen von Knochen.

### Stand der Technik

Die Form der Nase ist für ein attraktives Gesichtsprofil von zentraler Bedeutung. Idealerweise ist die Nase harmonisch verlaufend in den Gesichtszügen eingebettet. Weist die Nase jedoch eine eigenwillige Form und/oder eine überproportionale Grösse auf, so wird die Nase unweigerlich zum Blickfang und dadurch zum dominanten Teil des Gesichtes.

Die Form der Nase kann heute üblicherweise durch einen verhältnismäßig kleinen, aber dennoch sehr anspruchsvollen chirurgischen Eingriff dauerhaft korrigiert werden. In über 80 Prozent der Eingriffe wird die Nase verkleinert, in den übrigen die Nase vergrößert.

Im Wesentlichen besteht die Nase aus knöchernen und knorpeligen Anteilen, die ein pyramidenförmiges Gerüst unter der bedeckenden Nasenhaut bilden. Senkrecht in der Mitte befindet sich die Nasenscheidewand, die im vorderen Anteil knorpelig, im hinteren Anteil knöchern ist. Bei Nasenoperationen gilt es, dieses Gerüst so zu gestalten, dass sich die äußere Form der Nase durch die bedeckende Haut verändert. Die Rhinoplastik kann die Nase verkleinern oder vergrößern, die Form des Nasenrückens sowie des Naseneinganges mit den Nasenflügeln und den Nasenlöchern korrigieren, sowie die Länge der Nase und den Winkel zwischen Nase und Oberlippe verändern. Der Eingriff wird insbesondere aus ästhetischen Gründen zur Harmonisierung der Nase und des gesamten Gesichtes (ästhetische Rhinoplastik) durchgeführt. Damit der Eingriff keine sichtbaren Spuren hinterlässt, werden die Hautschnitte im Naseninneren oder am Nasensteg angelegt. Durch diesen Zugang modelliert der Arzt das Knochen- und Knorpelgerüst der Nase je nach Zielsetzung vorteilhaft um. So kann die Nase zum Beispiel verkleinert, verschmälert oder begradigt werden. Eine derartige Korrektur der Nase sollte erst nach Abschluss des Knochenwachstums, also etwa ab dem 16. oder 17. Lebensjahr vorgenommen werden.

Dieses bekannte Verfahren weist die Nachteile auf, dass ein anspruchsvoller chirurgischer Eingriff mit all den damit verbundenen Risiken erforderlich ist, dass der Eingriff sehr teuer ist und üblicherweise von den Krankenkassen nicht finanziert wird, und dass das Verfahren aus finanziellen Gründen nur einer begrenzten Bevölkerung zugänglich ist.

### Darstellung der Erfindung

Aufgabe der Erfindung ist es, eine Vorrichtung, sowie ein Verfahren zum Herstellen der Vorrichtung vorzuschlagen, welche ein kostengünstiges und sicheres Umformen von Knochen, und insbesondere eine Korrektur der Nasenform ermöglicht.

Diese Aufgabe wird gelöst mit einer Vorrichtung zum Umformen von Knochen oder Knorpel aufweisend die Merkmale von Anspruch 1. Die Unteransprüche 2 bis 12 betreffen weitere, vorteilhafte Ausgestaltungen. Die Aufgabe wird weiter gelöst mit einem Verfahren zum Herstellen der erfindungsgemässen Vorrichtung aufweisend die Merkmale von Anspruch 13.

Die Aufgabe wird insbesondere gelöst mit einer Vorrichtung zum Umformen von Knochen beziehungsweise zum Beeinflussen des Wachstums von Knochen oder Knorpel, insbesondere Nasenknochen, umfassend eine Formvorrichtung mit einer zur Auflage auf die Haut bestimmten Anpressfläche, und umfassend eine Haltevorrichtung, welche mit der Formvorrichtung verbindbar ist, wobei die Haltevorrichtung sowie die Formvorrichtung derart zusammenwirkend ausgestaltet ist, dass die Formvorrichtung zumindest an der Anpressfläche unter Ausübung einer Anpresskraft an der Haut anliegen kann.

Unter Knochen werden in der vorliegenden Schrift auch knochenähnliche Strukturen oder Knorpel verstanden.

Es hat sich herausgestellt, dass der Knochen des Menschen während des gesamten Lebens einem ständigen Umbauprozess unterworfen ist. Beim normalen Umbau des Knochens besteht ein Gleichgewicht zwischen osteoklastischer und osteoblastischer Aktivität. Eine dauernde Belastung des Knochens führt jedoch zu Anpassungsvorgängen wie Corticalisverdickung und Ausrichtung der Spongiosabälkchen. Wird die individuelle Belastungstoleranz durch wiederholte submaximale gleichartige Reize überschritten, finden im Bereich mechanischer Spannungsspitzen Strukturveränderungen und Umbauvorgänge statt. Die vorliegende Erfindung macht sich diesen Effekt zu nutzen, indem auf gewisse Knochenbereiche ein derartiger Druck erzeugt wird, sodass in diesem Knochen eine Spannung entsteht, welche die Ausformung des Knochens oder Knorpels beeinflusst. Dazu wird auf die Haut, welche sich über dem genannten Knochenbereich befindet, eine Kraft ausgeübt, welche sich über die Haut auf den Knochen fortpflanzt, sodass mit der erfindungsgemässen Formvorrichtung die Form des Knochens beeinflusst wird. Die erfindungsgemässe Vorrichtung wird vorzugsweise bei sich im Wachstum befindlichen Kindern verwendet, da die Ausgestaltung des Knochens, insbesondere des Nasenknochens, in dieser Phase relativ einfach beeinflussbar ist. Die erfindungsgemässe Vorrichtung ist jedoch auch für erwachsene Menschen verwendbar. Die erfindungsgemässe Vorrichtung wird vorzugsweise während mehreren Stunden pro Tag angewendet, beispielsweise während des Schlafs.

Um beispielsweise die Nasenform zu korrigieren oder umzuformen wird die erfindungsgemässe Vorrichtung vor dem Schlafen gehen montiert, indem die Formvorrichtung auf die Nase gelegt wird, und die Formvorrichtung mit einer Haltevorrichtung am Kopf fixiert wird. Die Formvorrichtung weist Anpressflächen auf, welche derart ausgerichtet, angeordnet und ausgestaltet sind, dass während dem Schlafen auf eine vorherbestimmte Stelle des Nasenknochens eine Presskraft ausgeübt wird.

Durch üblicherweise mehrmonatiges bis jahrelanges Tragen der erfindungsgemässen Vorrichtung während des Schlafens verändert sich die Form des Nasenknochens, beziehungsweise der wachsende Nasenknochen gleicht sich einer vorgegebenen Form an. Dies wird vorzugsweise während der Wachstumsphase angewendet, damit der Nasenknochen nach Abschluss des Knochenwachstums, das heisst nach dem 16. bis 17. Lebensjahr, die gewünscht Nasenknochenform aufweist und diese auch beibehält.

Die erfindungsgemässe Vorrichtung wird nachfolgend am Beispiel der Umformung der Nase im Detail erläutert. Die erfindungsgemässe Vorrichtung ist jedoch zur Umformung beinahe jedes Knochens des Menschen geeignet, beispielsweise auch zur Umformung des Kinns oder der Backenknochen. Die Erfindung wird nachfolgend an Hand von Ausführungsbeispielen beschreiben.

### Kurze Beschreibung der Zeichnungen

Die zur Erläuterung der Ausführungsbeispiele verwendeten Zeichnungen zeigen:
- Fig. 1: eine montierte Vorrichtung zum Umformen von Knochen;
- Fig. 2: die in Figur 1 dargestellte Vorrichtung im Detail;
- Fig. 3: einen Längsschnitt durch die in Figur 2 im Schnitt dargestellte, einstückige Formvorrichtung;
- Fig. 4: ein weiteres Ausführungsbeispiel einer Formvorrichtung;
- Fig. 5: einen Längsschnitt durch die in Figur 4 dargestellte und auf eine Nase aufgesetzte Formvorrichtung;
- Fig. 6: ein weiteres Ausführungsbeispiel einer Formvorrichtung;
- Fig. 7: ein weiteres Ausführungsbeispiel einer Formvorrichtung;
- Fig. 8: ein Ausführungsbeispiel eines Anpresskörpers;
- Fig. 9: ein weiteres Ausführungsbeispiel einer Vorrichtung zum Umformen von Knochen;
- Fig. 10: ein weiteres Ausführungsbeispiel eines Anpresskörpers;
- Fig. 11: ein weiteres Ausführungsbeispiel eines Anpresskörpers;
- Fig. 12: ein weiteres Ausführungsbeispiel eines Trägers;
- Fig. 13: ein Detail des in Figur 12 dargestellten Trägers;
- Fig. 14: ein weiteres Ausführungsbeispiel eines Trägers mit daran befestigtem Anpresskörper;
- Fig. 15: ein weiteres Ausführungsbeispiel eines Anpresskörpers;
- Fig. 16: ein weiteres Ausführungsbeispiel einer Formvorrichtung;
- Fig. 17: einen Querschnitt durch ein weiteres Ausführungsbeispiel eines Anpresskörpers;
- Fig. 18: eine Vorrichtung zum Herstellen der Vorrichtung zum Umformen von Knochen.

Grundsätzlich sind in den Zeichnungen gleiche Teile mit gleichen Bezugszeichen versehen.

### Wege zur Ausführung der Erfindung

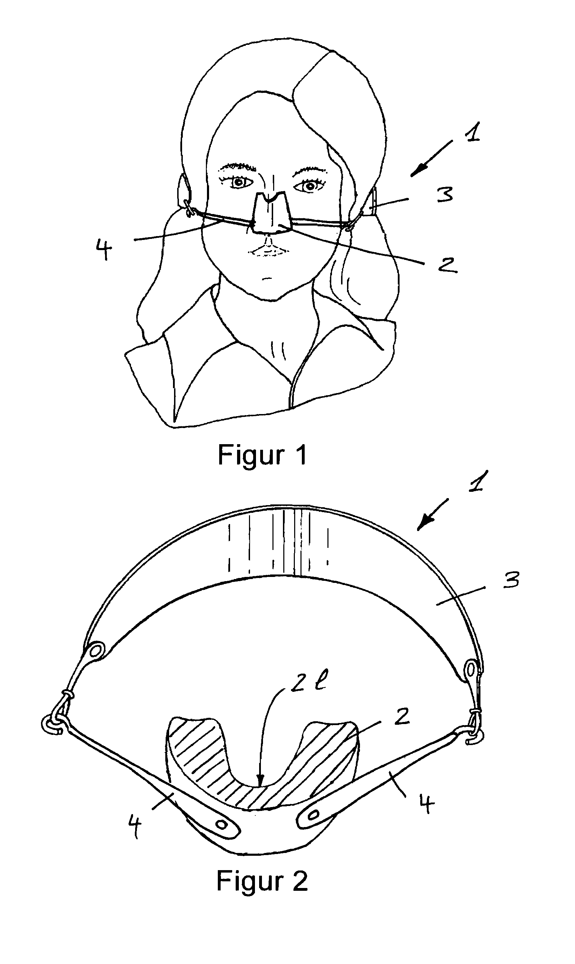

Fig. 1 zeigt eine am Kopf eines Teenagers fixierte Vorrichtung 1 zum Umformen von Nasenknochen beziehungsweise zur Beeinflussung der Form der Nase. Die Vorrichtung umfasst eine Formvorrichtung 2, welche über ein Verbindungsmittel 4 mit einer hinten um den Kopf verlaufenden Haltevorrichtung 3 verbunden ist. Diese Vorrichtung 1 wird üblicherweise über Nacht am Kopf fixiert, sodass die Formvorrichtung 2 während beispielsweise etwa acht Stunden eine Anpresskraft auf die Haut und den darunter liegenden Nasenknochen ausübt.

Figur 2 zeigt die in Figur 1 dargestellte Vorrichtung 1 im Detail. Die Formvorrichtung 2 ist in einem Querschnitt dargestellt, sodass die zur Aufnahme der Nase bestimmte Ausnehmung 21 ersichtlich ist. Die Formvorrichtung 2 ist einstückig ausgestaltet und besteht somit aus einem einzigen Teil. Die Haltevorrichtung 3 ist vorzugsweise elastische und die Verbindungsmittel 4 sind vorzugsweise starr ausgestaltet, damit die Formvorrichtung 2 mit genügend grosser Kraft gegen die Nase gepresst wird und verrutschfest bleibt, insbesondere während dem Schlaf. Die Haltevorrichtung 3 kann auf unterschiedlichste Weise ausgestaltet sein, um am Kopf befestigt zu werden. Beispielsweise kann die Haltevorrichtung 3 auch zwei oder drei hinter dem Kopf verlaufende Bänder aufweisen, um einen besseren Halt zu bewirken.

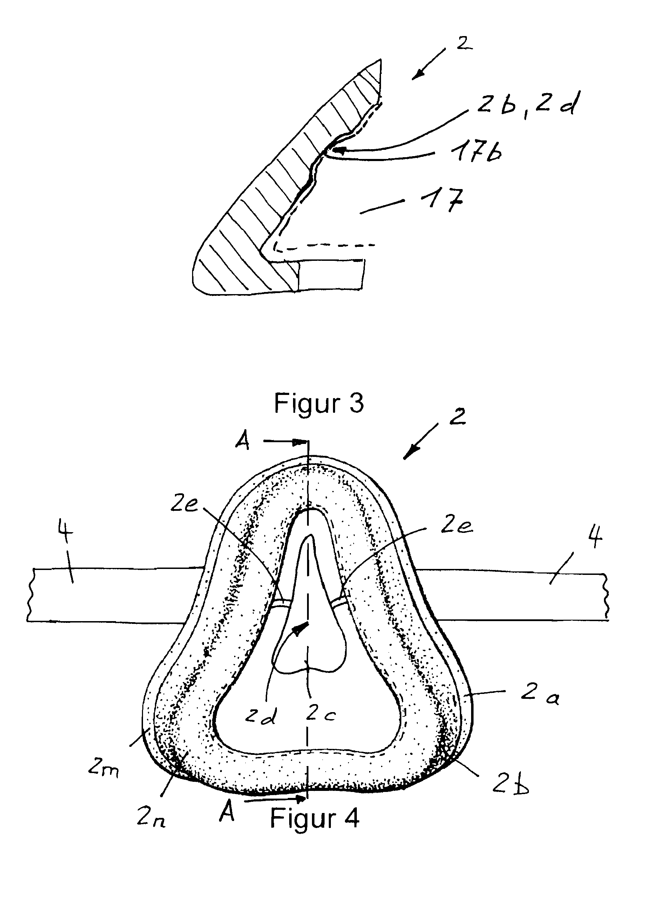

Figur 3 zeigt einen Längsschnitt durch die in Figur 2 dargestellte Formvorrichtung 2. Nur schematisch und gestrichelt ist der Verlauf der Nase 17 dargestellt. Die Nase weist einen Höcker 17b auf. Die Formvorrichtung 2 weist zumindest eine zur Auflage auf die Haut bestimmte Auflagefläche 2b mit Anpressfläche 2d auf, sodass die von der Formvorrichtung 2 ausgeübte Anpresskraft vorzugsweise zum grossen Teil über die Anpressfläche 2d erfolgt. Die Auflagefläche 2b ist daher derart bezüglich Ausrichtung und/oder Grösse und/oder Oberflächenverlauf ausgestaltet, dass die über die Anpressfläche 2d erzeugte Kraft auf eine bestimmte Hautstelle und somit auf einen vorherbestimmten Knochenbereich 17b einwirkt, um dessen Form und/oder Wachstum zu beeinflussen. Die Anpressfläche 2d kann auf unterschiedlichste Weise angeordnet und ausgestaltet sein, und beispielsweise auch aus zwei oder mehreren Teilflächen bestehen, oder beispielsweise entlang der gesamten Länge des Nasensattels oder entlang der Seitenkonturen verlaufen. Die Anordnung, Anzahl, Grösse und Ausrichtung der Anpressflächen 2d ist bestimmt durch die Lage der Knochen- beziehungsweise Knorpelbereiche, deren Form und/oder Wachstum beeinflusst werden sollten. Daher ist es auch wichtig, dass die Formvorrichtung 2 während vorzugsweise einer grösseren Anzahl Anwendungen reproduzierbar gleich oder ähnlich angeordnet auf die Nase aufgesetzt werden kann, damit die Anpresskraft auf dieselben Knochenbereiche ausgeübt wird. Daher kann es sich als vorteilhaft erweisen, die Formvorrichtung 2 mit einer derartig ausgestalteten Ausnehmung 21 zu versehen, dass die Formvorrichtung 2 quasi selbst zentrierend auf die Nase aufgesetzt werden kann. Die Formvorrichtung 2 ist beispielsweise aus Kunststoff gefertigt, wobei die Formvorrichtung 2 auch lokal unterschiedliche Elastizitäten aufweisen kann.

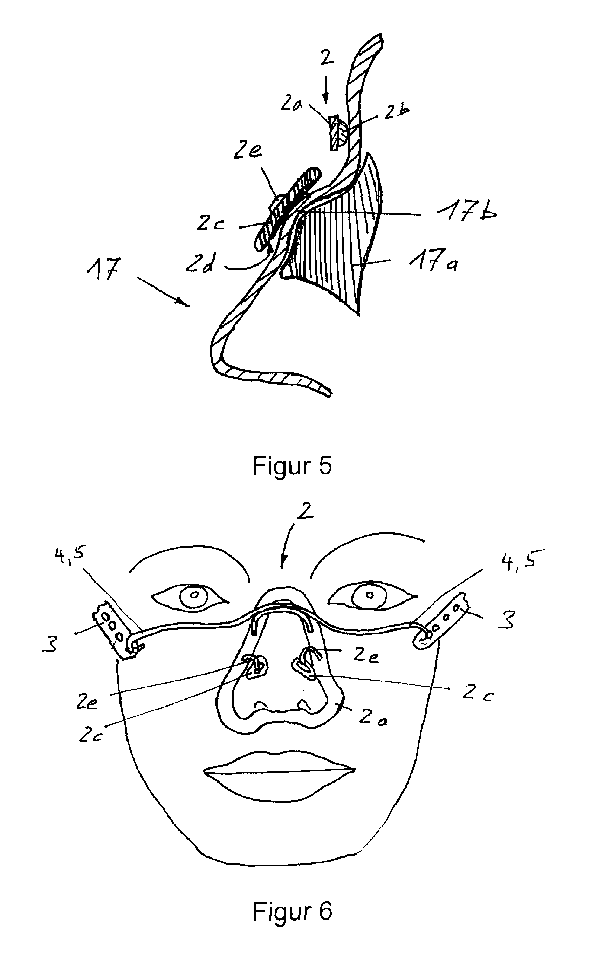

In einer vorteilhaften Ausgestaltung umfasst die Formvorrichtung 2, wie in Figur 4 dargestellt, einen Träger 2a mit anatomisch ausgestalteter Auflagefläche 2b, wobei der Träger 2a mit den Verbindungsmitteln 4 verbunden ist. Der Träger 2a besteht beispielsweise aus einem metallischen Rahmen 2m sowie einem elastischen Kunststoff 2n, welcher die Auflagefläche 2b ausbildet. Im dargestellten Ausführungsbeispiel entspricht der Verlauf der Innenkontur des Trägers 2a dem Verlauf der Aussenkontur der Nasenwurzel, sodass der Träger 2a derart am Gesicht angelegt werden kann, dass der Träger 2a die Nasen umschliesst und im Bereich der Nasenwurzel über die Auflagefläche 2b am Gesicht anliegt. Dieses grossflächige und durch die Lage der Nase bestimmte Anlegen des Trägers 2a weist den Vorteil auf, dass der Träger 2a reproduzierbar gleich und verrutschfest oder im Wesentlichen verrutschfest, ähnliche wie in Figur 1 dargestellt, angelegt werden kann. Der Träger 2a beziehungsweise dessen Metallrahmen 2m bildet somit eine Basisteil, an welchem ein oder mehrere Anpresskörper 2c befestigt werden können. Im dargestellten Ausführungsbeispiel ist ein Anpresskörper 2c über zwei als Stege ausgebildete Haltemittel 2e fest mit dem Träger 2a verbunden. Der Anpresskörper 2c weist eine Anpressfläche 2d auf. Es könnten auch mehrere Anpresskörper 2c mit dem Träger 2a verbunden sein. Die Anpresskörper 2c könnte auch nur mit einem einzigen Haltemittel 2e mit dem Träger 2a verbunden sein. Die Anpresskörper 2c könnten bezüglich dem Haltemittel 2e auch verschiebbar und/oder lösbar und wieder fixierbar angeordnet sein. Der in Figur 4 dargestellte Träger 2a kann somit auf unterschiedlichste Weise mit Anpresskörpern 2c bestückt werden.

Figur 5 zeigt schematisch einen Längsschnitt durch eine Nase 17 mit Nasenknochen 17a. Auf die Nase 17 ist die in Figur 4 dargestellte Formvorrichtung 2 aufgesetzt. Der Anpresskörper 2c mit Anpressfläche 2d liegt am Nasenhöcker 17b an, und übt eine auf den Nasenhöcker 17b beziehungsweise auf den Nasenknochen 17a wirkende Anpresskraft aus. Der Anpresskörper 2c beziehungsweise die Anpressfläche 2d kann, wie bereits mit Figur 3 beschrieben, abhängig von der jeweiligen anatomischen Form der Nase, in einer Vielzahl von Möglichkeiten ausgestaltet und angeordnet sein. Es können auch mehrere Anpressflächen 2d oder auch eine Mehrzahl von Anpresskörper 2c vorgesehen sein.

Figur 6 zeigt schematisch eine Formvorrichtung 2 mit einem Träger 2a, an welchem zwei Anpresskörper 2c angeordnet sind, wobei jeder Anpresskörper 2c über ein Haltemittel 2e mit dem Träger 2a fest verbunden ist. In einer bevorzugten Ausgestaltung sind die Anpresskörper 2c lösbar mit dem jeweiligen Haltemittel 2e verbunden, sodass die Anpresskörper 2c ausgetauscht werden können. Der Träger 2a ist mit starren Verbindungsmitteln 4 beziehungsweise über einen Gesichtsbogen 5 mit der Haltevorrichtung 3 verbunden.

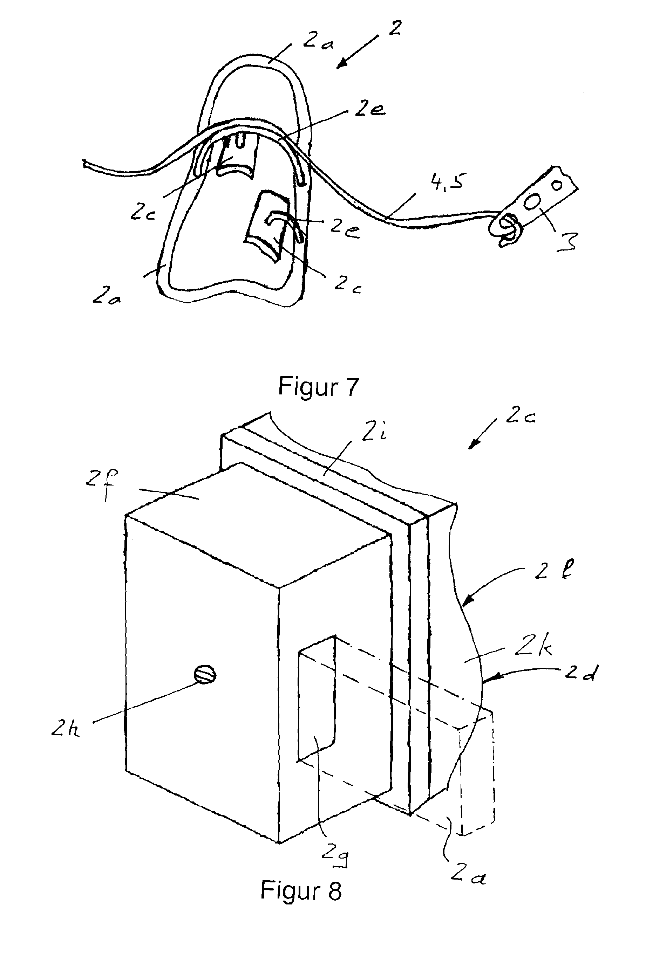

Figur 7 zeigt ein weiteres Ausführungsbeispiel einer Formvorrichtung 2 mit Träger 2a und zwei Anpresskörpern 2c. Ein u-förmig ausgestaltetes Haltemittel 2e ist an beiden Enden mit dem Träger 2a verbunden, wobei ein Anpresskörper 2c am u-förmigen Haltemittel 2e befestigt ist. Das Haltemittel 2e ist über ein als Gesichtsbogen 5 ausgestaltetes Verbindungsmittel 4 mit der Haltevorrichtung 3 verbunden. Die gesamte Formvorrichtung 2 ist somit über den Gesichtsbogen 5 mit der Haltevorrichtung 3 verbunden. Unter Gesichtsbogen 5 wird ein starrer, gekrümmt verlaufender Draht verstanden, welche die Kraft von der Formvorrichtung 2 zur Haltevorrichtung 3 überträgt. Der Gesichtsbogen 5 ist vorzugsweise derart gekrümmt beziehungsweise gewölbt verlaufend ausgestaltet, dass dieser nicht an der Haut anliegt, und somit die Formvorrichtung 2 mit der Haltevorrichtung 3 verbinden, ohne die Haut zu berühren.

In einem weiteren, nicht dargestellten Ausführungsbeispiel könnte die Formvorrichtung 2 gleichzeitig sowohl über das in Figur 6 dargestellte Verbindungsmittel 4 als auch über den in Figur 7 dargestellten Gesichtsbogen 5 mit der Haltevorrichtung 3 verbunden sein.

Figur 8 zeigt ein Ausführungsbeispiel eines Anpresskörpers 2c im Detail. Der Anpresskörper 2c umfasst ein Basisteil 2f mit einer Ausnehmung 2g, wobei, wie gestrichelt angedeutet, ein Träger 2a beziehungsweise ein Haltemittel 2e durch die Ausnehmung 2g verläuft, sodass der Anpresskörper 2c bezüglich dem Träger 2a beziehungsweise dem Haltemittel 2e verschiebbar gelagert ist, und mit einer Schraube 2h fixiert werden kann. Der Anpresskörper 2c umfasst eine Basisplatte 2i, auf welcher ein Formteil 2k angeordnet ist. Das Formteil 2k weist auf der der Basisplatte 2i gegenüberliegenden Seite eine Oberfläche 21 auf, welche vorzugsweise dreidimensional verlaufend ausgestaltet ist, wobei die Oberfläche 21 zumindest eine definierte Anpressfläche 2d ausbildet, welche in Figur 8 angedeutet ist. Das Formteil 2k ist vorzugsweise aus einem hautfreundlichen Material, beispielsweise einem Kunststoff gefertigt.

Das in Figur 8 dargestellte Ausführungsbeispiel eines Anpresskörpers 2c weist den Vorteil auf, dass der Verlauf der Oberfläche 21 individuell ausgestaltet werden kann, wogegen die übrigen Teile des Anpresskörpers 2c als identisch ausgestaltete Standardteile ausgebildet sein können. Ebenso ist es möglich die Anpresskörper 2c in wenigen unterschiedlichen Grössen, zum Beispiel in drei bis vier Einheitsgrössen zu fertigen, sodass eine Formvorrichtung 2 mit Hilfe eines Trägers 2a und einer Mehrzahl der standardisierten Anpresskörper 2c zusammengebaut werden kann. Jeder Anpresskörper 2c wird vorzugsweise mit einem den individuellen Bedürfnissen angepasst verlaufenden Formteil 2k beziehungsweise mit einer individuell verlaufenden Oberfläche 21 versehen.

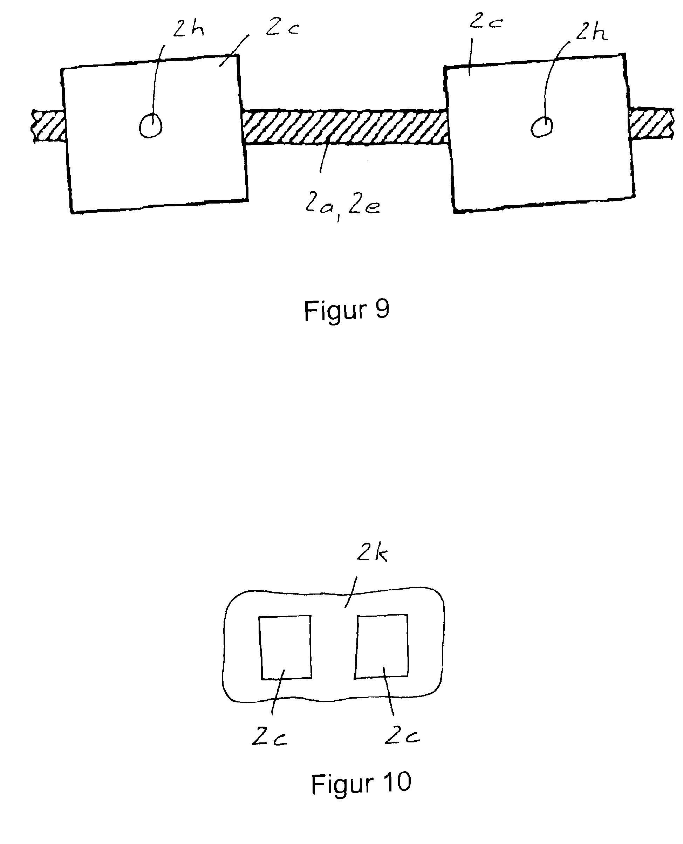

Figur 9 zeigt im Detail, wie die in Figur 8 dargestellten Anpresskörper 2c an einem Haltemittel 2e, beispielsweise der in Figur 6 dargestellten Formvorrichtung 2 befestigt werden könnten. Figur 9 zeigt schematisch ein Haltemittel 2e, an welchem die zwei Anpresskörper 2c mit Hilfe von Schrauben 2h fest fixiert sind.

Figur 10 zeigt schematisch ein weiteres Formteil 2k, welches im Unterschied zu dem in Figur 8 dargestellten Ausführungsbeispiel, mit zwei Anpresskörpern 2c verbunden ist.

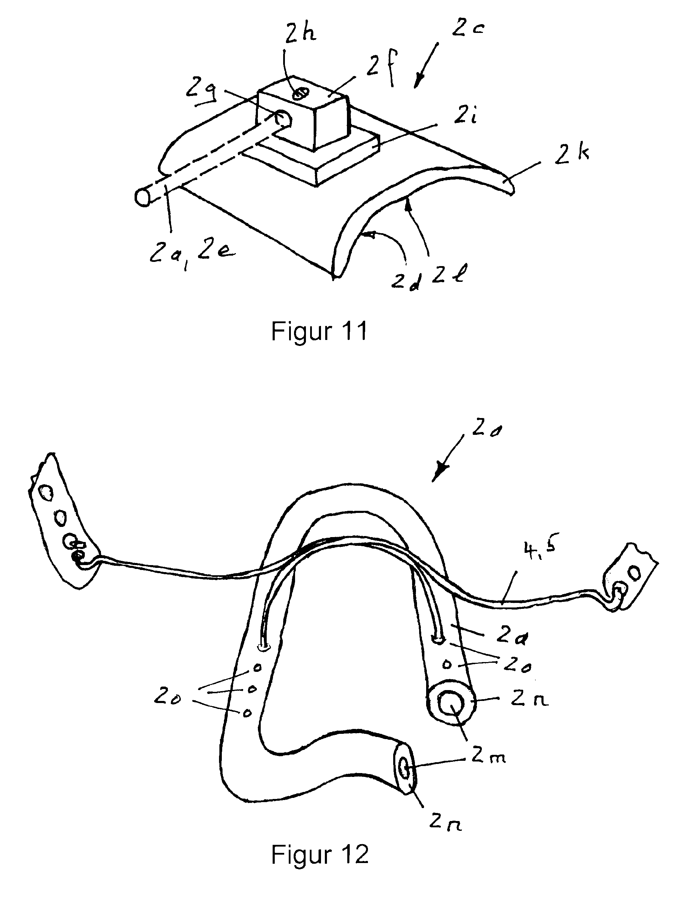

Figur 11 zeigt ein weiteres Ausführungsbeispiel eines Anpresskörpers 2c. Der Anpresskörper 2c umfasst ein Basisteil 2f mit einer Ausnehmung 2g, in welche ein Haltemittel 2e hineinragt. Das Haltemittel 2e ist mit Hilfe der Schraube 2h lösbar mit dem Basisteil 2f verbunden. Der Anpresskörper 2c umfasst zudem ein Formteil 2k, welches über die Basisplatte 2i mit dem Basisteil 2f verbunden ist.

Das Formteil 2k weist eine Oberfläche 21 auf, welche eine Anpressfläche 2d ausbildet.

Figur 12 zeigt ein weiteres Ausführungsbeispiel eines Trägers 2a mit einem runden, metallischen Rahmen oder einem Kunststoffrahmen 2m sowie einem den Rahmen 2m umschliessenden elastischen Kunststoff 2n. Der metallische Rahmen oder der Kunststoffrahmen 2m kann in einer vorteilhaften Ausführungsform als biegsam ausgestaltet sein, sodass der Verlauf des Rahmens 2m beispielsweise dem anatomischen Verlauf der Nase beziehungsweise der Nasenwurzel entsprechend angepasst werden kann. Jeder Schenkel des Trägers 2a ist über je einen Gesichtsbogen 5 mit der Haltevorrichtung 3 verbunden. Die beiden Gesichtsbögen 5 sind an deren Kreuzungsstelle fest miteinander verbunden.

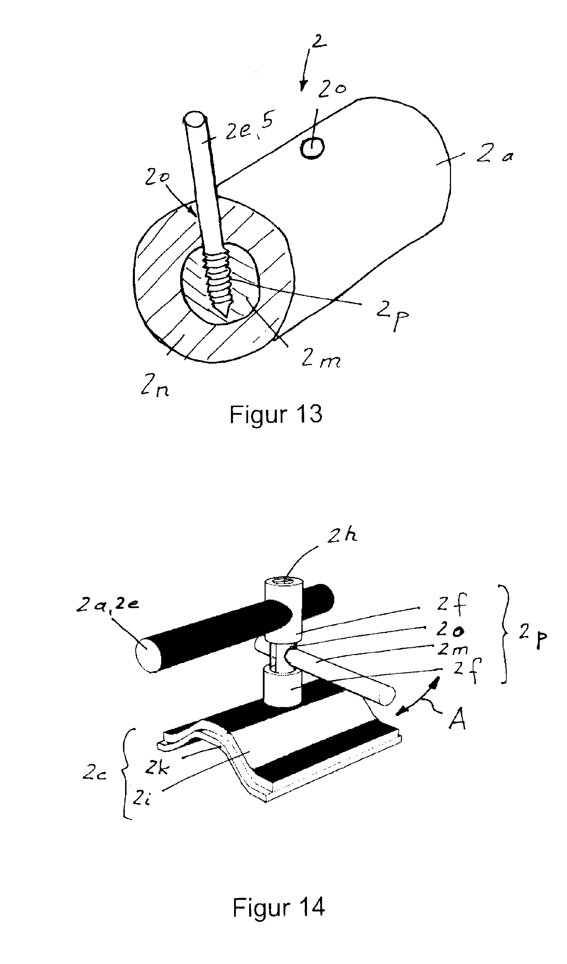

Figur 13 zeigt in einer Detailansicht, wie ein Haltemittel 2e beziehungsweise ein Gesichtsbogen 5 mit dem Träger 2a verbindbar ist. Der Träger 2a weist im Kunststoff 2n ein Loch 2o auf, welches in ein Innengewinde 2p des Rahmens 2m münden. Das Haltemittel 2e beziehungsweise der Gesichtsbogen 5 weist ein Aussengewinde auf, welches fest mit dem Innengewinde 2p verschraubbar ist, sodass das Haltemittel 2e fest und lösbar mit dem Träger 2a verbunden ist. Anstelle eines Gewindes könnte die Verbindung von Rahmen 2m und Haltemittel 2e auch über eine Steck- oder Schnappverbindung erfolgen. Der Träger 2a kann eine Mehrzahl derartiger Löcher 2o aufweisen, über welche ein Anpresskörper 2c, beispielsweise der in Figur 11 dargestellte Anpresskörper 2c, mit dem Träger 2a verbindbar ist. Diese Mehrzahl von Löchern 2o weist, wie in Figur 12 dargestellt, den Vorteil auf, dass der Anpresskörper 2c in einer Vielzahl möglicher Positionen bezüglich dem Träger 2a beziehungsweise bezüglich der Nase 17 befestigbar ist. Zudem ist es möglich eine Mehrzahl von Anpresskörpern 2c fest mit einem Träger 2a zu verbinden. In vorzugsweise gegenüberliegenden Löchern 2o könne beispielsweise auch, wie in Figur 7 dargestellt, ein u-förmiges Haltemittel 2e verankert werden. Es könnten auch eine Mehrzahl in Längsrichtung der Nase beabstandet angeordnete Haltemittel 2e mit dem Träger 2a verbunden sein.

In einer vorteilhaften Ausgestaltung steht beim Anpassen der Formvorrichtung 2 ein Bausatz beziehungsweise ein Kit zur Verfügung, welcher eine Mehrzahl von Trägern 2a unterschiedlicher Grösse und/oder Gestalt, sowie eine Mehrzahl von Anpresskörpern 2c, vorzugsweise auch unterschiedlicher Grösse und/oder Gestalt, sowie eine Mehrzahl unterschiedlicher Gesichtsbogen 5 umfasst. Ein derartiger Bausatz ermöglicht es eine Vielzahl unterschiedlich ausgestalteter, individuell an die Anatomie eines Menschen angepasste Formvorrichtungen 2 zu bilden.

Figur 14 zeigt ein weiteres Ausführungsbeispiel eines Anpresskörpers 2c, welcher über ein Haltemittel 2p mit einem Träger 2a oder einem Haltemittel 2e verbunden ist. Der Anpresskörper 2c umfasst eine vorgeformte Basisplatte 2i aus vorzugsweise festem Material wie Metall oder Kunststoff, sowie eine weiches Formteil 2k, beispielsweise aus Schaumstoff. Das Haltemittel 2p umfasst zwei Basisteile 2f, wobei das eine Basisteil 2f mit Hilfe der Schraube 2h fest und lösbar mit dem Träger 2a oder dem Haltemittel 2e verbunden ist. Nach dem Lösen der Schraube 2h kann das Basisteil 2f entlang des Trägers 2a beziehungsweise des Haltemittels 2e verschoben werden. Das in Figur 14 unten angeordnete Basisteil 2f ist fest mit der Basisplatte 2i verbunden. Die beiden Basisteile 2f weisen entgegengesetzt verlaufende Innengewinde auf, in welchen ein Distanzeinstellgewinde 2o verdrehbar gelagert ist, sodass die Distanz zwischen den beiden Basisteilen 2f durch Drehen des Distanzeinstellgewindes 20 vergrösserbar oder verkleinerbar ist. Um das Distanzeinstellgewinde 20 zu verdrehen kann, wie dargestellt, ein Schlüssel 2m in ein Loch des Distanzeinstellgewindes 2o eingeführt und anschliessend in Bewegungsrichtung A gedreht werden, um dadurch die Gesamtlänge des Haltemittel 2p zu vergrössern oder zu verkleinern, beziehungsweise um dadurch die auf die Nase wirkende Anpresskraft zu vergrössern oder zu verkleinern. Der Schlüssel 2m wird vorzugsweise nur zum Verstellen des Haltemittels 2p in das Distanzeinstellgewinde 2o eingeführt, und danach wieder vollständig entfernt. Die Verwendung eines derartigen Schlüssel 2m weist den Vorteil auf, dass dieser relativ lang ausgestaltet sein kann, sodass mit Hilfe dieses Schlüssels 2m ein relativ grosses Drehmoment auf das Distanzeinstellgewinde 2o bewirkt werden kann, sodass der Anpresskörper 2c, falls erforderlich, auch mit einer hohen Anpresskraft versehen werden kann.

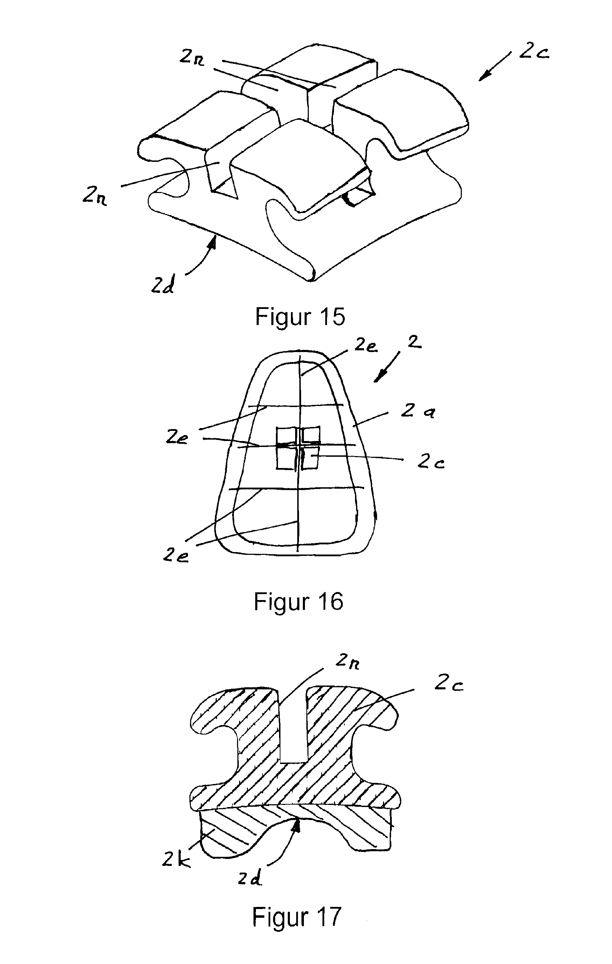

Am Träger 2a könnten auch, wie in Figur 16 in einer Draufsicht dargestellt, eine Mehrzahl von Haltemitteln 2e angeordnet sein, welche lösbar oder fest mit dem Träger 2a verbunden sind. Die Haltemittel 2e können auch gitterförmig ausgestaltet sein, mit zum Beispiel fest verbundenen Kreuzungspunkten. Die Haltemittel 2e sind vorteilhafterweise als dünner Draht von beispielsweise 1 mm, oder 0.5 mm bis 1.5 mm, Durchmesser ausgestaltet, wobei das Gitter derart gewölbt ist, dass dieses bei aufgesetzter Formvorrichtung 2 bezüglich der Nase beabstandet verläuft. Ein derartiger Träger 2a mit Haltemittel 2e kann ein sehr geringes Gewicht aufweisen. Vorteilhafterweise wird, wie in Figur 15 dargestellt, ein Anpresskörper 2c verwendet, der sich kreuzende Nuten 2n aufweist. Dieser Anpresskörper 2c wird, wie in Figur 16 dargestellt, vorteilhafterweise an sich kreuzenden Haltemitteln 2e mit diesen verbunden, z.B. durch Löten oder Kleben, sodass der Anpresskörper 2c in einer genau definierten Lage im Gitter beziehungsweise in der Formvorrichtung 2 gehalten ist. Falls erforderlich kann der Anpresskörper 2c auch durch einen anderen Anpresskörper 2c ersetzt werden. Es wäre jedoch auch ein Anpresskörper 2c ohne kreuzende Nuten geeignet, welcher am Gitter befestigt werden kann. Es könnten auch eine Mehrzahl von Anpresskörper 2c am Gitter befestigt werden.

Figur 17 zeigt einen Querschnitt durch ein weiteres Ausführungsbeispiel eines Anpresskörpers 2c. Im Unterschied zu dem in Figur 15 dargestellten Ausführungsbeispiel weist der Anpresskörper 2c gemäss Figur 17 zusätzlich ein Formteil 2k auf, welche die Anpressfläche 2d ausbildet.

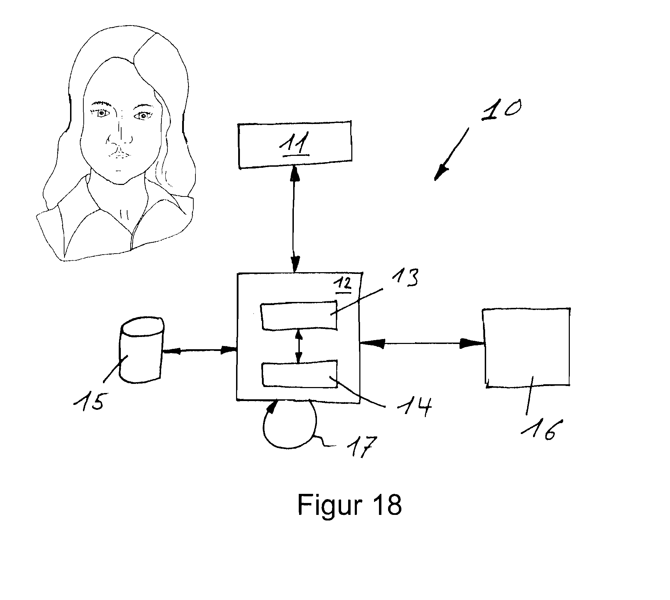

Figur 18 zeigt schematisch eine Vorrichtung 10 zum Herstellen der Formvorrichtung 2. Im dargestellten Ausführungsbeispiel wird die Erstellung einer Formvorrichtung 2 zum Umformen der Nase dargestellt. Dieselbe Vorrichtung 10 ist natürlich auch zum Erstellen von Formvorrichtungen 2 für andere Körperteile geeignet. Die Form der Nase wird mit einem 3-dimensionalen Scanner 11 abgetastet und die Daten einem Server 12 übermittelt. Im Speicher 14 wird ein 3-dimensionales Datenmodell der ausgemessenen Form der Nase bespeichert. Daraufhin kann mit Hilfe eines interaktiven Verfahrens 17 ein 3-dimensionales Datenmodell der gewünschten Nasenform gespeichert werden. Der Server hat Zugriff auf eine Datenbank 15, in welcher die Daten einer Mehrzahl von Formvorrichtungen 2, Trägern 2a, Anpresskörper 2c usw. gespeichert sind. Das im Server 12 gespeicherte Verarbeitungsmodul 13 ist in der Lage, basierend auf den genannten, zur Verfügung stehenden Daten den Aufbau einer geeigneten Formvorrichtung 2 vorzuschlagen. Die gesamte Formvorrichtung 2 kann beispielsweise basierend auf Standartkomponenten zusammengestellt werden. In einer bevorzugten Ausgestaltung wird der Oberflächenverlauf des Formteils 2k jedes Anpresskörpers 2c individuell ausgestaltet, indem beispielsweise der Server 12 mit einer Maschine 16 verbunden ist, welche ein Formteil 2k mit individuell verlaufender Oberfläche 21 herzustellen erlaubt. In einer vorteilhaften Ausgestaltung sind die Anpresskörper 2c in ihrer Grundform mit einem konstant dicken Formteil 2k versehen. Dieses Formteil 2k kann in der Maschine 16 individuell bearbeitet werden, sodass das derart gefertigte Formteil 2k eine individuell verlaufende Oberfläche 21 aufweist, mit zumindest einer Anpressfläche 2d. Der zumindest eine Anpresskörper 2c wird daraufhin am Träger 2a befestigt, sodass die Formvorrichtung 2 ausgebildet wird, welche daraufhin mit einer Vorspannkraft an die Nase angelegt werden kann, um deren Form zu beeinflussen.

Das Verfahren zum Herstellen einer Formvorrichtung umfasst in einer bevorzugten Ausgestaltung die folgenden Schritte:
a) Berührungsloses Abtasten der zu verändernden Hautoberfläche und Erstellen eines digitalen Modells des Verlaufs der Hautoberfläche der zu verändernden Körperstelle;
b) Erhalten eines digitalen Modells des Verlaufs der darunter liegenden Knochen;
c) Festlegen des gewünschten Verlaufs der Hautoberfläche der Körperstelle nach der Veränderung;
d) Festlegen der zu verändernden Bereiche der Knochen;
e) Bestimmen von zumindest einer Anpressfläche 2d bezüglich mindestens eines Parameters aus der Gruppe Grösse, Ausrichtung, Oberflächenverlauf;
f) und Herstellen der Formvorrichtung 2 umfassend die Anpressfläche 2d.

## Patentansprüche

1. Vorrichtung (1) zum Umformen von Knochen, insbesondere Nasenknochen, umfassend:
eine Formvorrichtung (2) mit einer zur Auflage auf die Haut bestimmten Anpressfläche (2d),
und eine Haltevorrichtung (3), welche mit der Formvorrichtung (2) verbindbar ist,
wobei die Haltevorrichtung (3) sowie die Formvorrichtung (2) derart zusammenwirkend ausgestaltet ist, dass die Formvorrichtung (2) zumindest an der Anpressfläche (2d) unter Ausübung einer Anpresskraft an der Haut anliegen kann.

2. Vorrichtung nach Anspruch 1, **dadurch gekennzeichnet, dass** die Formvorrichtung (2) einen Träger (2a) umfasst, und dass der Träger (2a) zumindest einen Anpresskörper (2c) mit Anpressfläche (2d) aufweist.

3. Vorrichtung nach Anspruch 2, **dadurch gekennzeichnet, dass** der Träger (2a) eine Auflagefläche (2b) aufweist, welche derart ausgestaltet ist, dass diese grossflächig an der Haut anliegen kann.

4. Vorrichtung nach Anspruch 2 oder 3, **dadurch gekennzeichnet, dass** der Träger (2a) derart verlaufend ausgestaltet ist, dass diese der Aussenkontur der Nase folgend am Gesicht aufliegen kann.

5. Vorrichtung nach einem der Ansprüche 2 bis 4, **dadurch gekennzeichnet, dass** der Anpresskörper (2c) bezüglich dem Trägre (2a) verschiebbar und/oder ersetzbar und/oder fixierbar gelagert ist.

6. Vorrichtung nach einem der Ansprüche 2 bis 5, **dadurch gekennzeichnet, dass** der Träger (2a) zumindest ein Haltemittel (2e) umfasst, und dass der Anpresskörper (2c) mit dem Haltemittel (2e) verbunden ist.

7. Vorrichtung nach Anspruch 6, **dadurch gekennzeichnet, dass** das Haltemittel (2e) u-förmig ausgestaltet und an beiden Enden mit dem Träger (2a) verbunden ist.

8. Vorrichtung nach Anspruch 6 oder 7, **dadurch gekennzeichnet, dass** das Haltemittel (2e) gitterförmig ausgestaltet ist.

9. Vorrichtung nach einem der Ansprüche 2 bis 8, **dadurch gekennzeichnet, dass** der Anpresskörper (2c) ein Formteil (2k) umfasst, welches die Anpressfläche (2d) ausbildet.

10. Vorrichtung nach einem der vorhergehenden Ansprüche, **dadurch gekennzeichnet, dass** die Formvorrichtung (2) einstückig ausgebildet ist.

11. Vorrichtung nach einem der vorhergehenden Ansprüche, **dadurch gekennzeichnet, dass** die Anpressfläche (2d) derart in der Formvorrichtung (2) angeordnet und ausgestaltet ist, dass die Anpressfläche (2d) zumindest über einem Teilabschnitt entlang dem Nasensattel anliegt.

12. Vorrichtung nach einem der vorhergehenden Ansprüche, **dadurch gekennzeichnet, dass** die Formvorrichtung (2) über einen zusätzlichen Gesichtsbogen (5) mit der Haltevorrichtung (3) verbunden ist.

13. Verfahren zum Herstellen einer Vorrichtung nach einem der vorhergehenden Ansprüche, umfassend die Schritte:
a) Erhalten eines digitalen Modells des Verlaufs der Hautoberfläche der zu verändernden Körperstelle;
b) Erhalten eines digitalen Modells des Verlaufs der darunter liegenden Knochen;
c) Festlegen der zu verändernden Bereiche des Knochens;
d) Bestimmen von zumindest einer Anpressfläche (2d) bezüglich mindestens einem Parameter aus der Gruppe Grösse, Ausrichtung, Oberflächenverlauf;
e) und Erstellen der Formvorrichtung (2) umfassend die Anpressfläche (2d).

14. Bausatz zum Erstellen einer Formvorrichtung (2) nach einem der Ansprüche 1 bis 12, umfassend zumindest einen Träger (2a) und eine Mehrzahl unterschiedlich ausgestalteter Anpresskörper (2c), welche mit dem Träger (2a) verbindbar sind.
